# EUROPÄISCHE PATENTANMELDUNG

(11) **EP 4 066 793 A1**
(43) Veröffentlichungstag der Anmeldung: **05.10.2022**
(21) Anmeldenummer: 22164706.8
(22) Anmeldetag: 28.03.2022
(51) Int. Cl.: A61F 2/76, A61F 2/50

(54) **ADAPTERSYSTEM ZWISCHEN EINEM PROTHESENSTUMPFSCHAFT UND HANDELSÜBLICHEN MODULSYSTEMEN, ADAPTEREINRICHTUNG HIERFÜR, PROTHESENSTUMPFSCHAFT HIERFÜR UND PROTHESE AUFWEISEND DAS ADAPTERSYSTEM**

(30) Priorität: 30.03.2021 DE 102021108095
(71) Anmelder: OT Supply GmbH, 86529 Schrobenhausen (DE)
(72) Erfinder: SCHASER, Werner, 86529 Schrobenhausen (DE); DALLMAYER, Robert, 86529 Schrobenhausen (DE); KURZHALS, Christoph, 86529 Schrobenhausen (DE)
(74) Vertreter: Kuhnen & Wacker Patent- und Rechtsanwaltsbüro PartG mbB

(57) **Zusammenfassung**

Die Erfindung betrifft ein Adaptersystem zwischen einem Prothesenschaft (1) und handelsüblichen Modulsystemen aufweisend eine Anbindungsplattform (2), eine an der Anbindungsplattform (2) befestigbare Adaptereinrichtung (8), wobei wenigstens ein Modulsystemelement (12) wenigstens an der Adaptereinrichtung (8) befestigbar ist.

## Beschreibung

Die Erfindung betrifft ein Adaptersystem zwischen einem Prothesenschaft und handelsüblichen Modulsystemen, eine Adaptereinrichtung hierfür, einen Prothesenschaft hierfür und eine Prothese aufweisend das Adaptersystem.

Aus dem Stand der Technik, insbesondere aus der DE 197 18 580 A1 ist ein Schaftadapter zum Verbinden eines Schaftes mit einem Rohradapter bekannt. Der Schaftadapter weist ein Adapterelement mit einer Ausnehmung zur Aufnahme eines mit dem Schaft verbundenen Stiftes auf, wobei die Ausnehmung wesentlich größer ist als die Querschnittsabmessung des Stiftes und die Fixierung in jeder Position der Ausnehmung möglich ist, wodurch erreicht wird, dass mit einer seitlichen Anpassung die Trageeigenschaften wesentlich verbessert werden. Bei einem derartigen Schaftadapter ist von Nachteil, dass die Anbindung des Rohradapters, insbesondere dessen Positionierung bezüglich des einen Gliedmaßenstumpf aufnehmenden Schaftes bei der Herstellung einer Prothese besonders schwierig und aufwändig ist. Zudem ist der vorgeschlagene Schaftadapter besonders vielteilig und kompliziert in der Montage.

Des Weiteren ist es bekannt, eine Anbindungsplattform zur Aufnahme eines Modulsystemelements, wie z. B. eines Schaftadapters in den Prothesenschaft einzugießen oder einzulaminieren. Die Anbindungsplattform liegt dabei insbesondere in Form eines vorgefertigten Teils vor, welches beim Herstellprozess des Prothesenschaftes in entsprechende Gießmassen oder Laminatschichten eingebettet und so bezüglich des Prothesenschaftes befestigt wird.

Ein derartiges Eingießen oder Einlaminieren birgt eine oftmals beobachtete Fehlerquelle, insbesondere kann es zu unerwünschten Verschiebungen der Anbindungsplattform kommen, was nach Abschluss der Prothesenherstellung nahezu unmöglich, wenigstens aber sehr aufwändig, zu korrigieren ist.

Des Weiteren kann es zu ungewollten Verschmutzungen, beispielsweise von Öffnungen und/oder Gewinden mit Gießmasse oder Laminierharz kommen, so dass eine erhöhte Nacharbeit notwendig ist.

Aufgabe der Erfindung ist es daher, ein Adaptersystem zwischen einem Prothesenschaft und handelsüblichen Modulsystemen, wie z. B. eines Schaftadapters anzugeben, welche sich in besonderer Art und Weise zur einfachen Herstellung von Prothesenschäften zur Anbindung z. B. eines Rohradapters eignet.

Insbesondere ist es auch Aufgabe der Erfindung, eine Adaptereinrichtung hierfür anzugeben.

Aufgabe der Erfindung ist es ebenfalls, einen Prothesenschaft und eine Prothese aufweisend das Adaptersystem anzugeben.

Allen Einzellösungen gemeinsam ist, dass eine einfache Herstellbarkeit, insbesondere eine hochfeste und eine insbesondere im Vorfeld der Herstellung leicht korrigierbare Positionierung der Anbindungsplattform bezüglich des Prothesenschafes ermöglicht ist.

Das Adaptersystem soll universell bei einer Vielzahl von patientenindividuellen Prothesenschäften anwendbar sein. Das Adaptersystem und die Adaptereinrichtung sollen insbesondere eine feste, dauerhafte und widerstandsfähige Anbindung des Modulsystems, insbesondere des Modulsystemelements, wie z. B. des Schaftadapters, der beispielsweise mit einem umgekehrt pyramidalen Befestigungsvorsprung versehen ist, ermöglichen.

Die oben genannten Aufgaben werden mit einem Adaptersystem mit den Merkmalen des Anspruchs 1 gelöst. Vorteilhafte Ausführungsformen sind in den Unteransprüchen angegeben.

Des Weiteren wird die Aufgabe hinsichtlich der Adaptereinrichtung mit einer Adaptereinrichtung gemäß Anspruch 14 gelöst.

Hinsichtlich des Prothesenschaftes wird die Aufgabe mittels eines Prothesenschafts mit den Merkmalen des Anspruchs 15 gelöst.

Hinsichtlich der Prothese wird die Aufgabe mit einer Prothese mit den Merkmalen des Anspruchs 16 gelöst.

Bei einem erfindungsgemäßen Adaptersystem zwischen einem Prothesenschaft und einem handelsüblichen Modulsystem, insbesondere einem handelsüblichen Schaftadapter, ist vorhanden:
- eine Anbindungsplattform;
- eine an der Anbindungsplattform befestigbare Adaptereinrichtung, wobei
- wenigstens ein Modulsystemelement, z. B. ein Schaftadapter wenigstens an der Adaptereinrichtung befestigbar ist.

Mit dem erfindungsgemäßen Adaptersystem zwischen einem Prothesenschaft und einem handelsüblichen Modulsystem, insbesondere einem handelsüblichen Schaftadapter, werden die erfindungsgemäß gestellten Aufgaben in vorteilhafter Weise gelöst. Insbesondere können umständliche und handwerklich aufwändige Einlaminier- bzw. Eingießprozesse vermeidbar sein und somit entsprechende Prothesen besonders passgenau und flexibel an einem patientenindividuellen Versorgungsfall anpassbar sein.

Gemäß einer vorteilhaften Ausführungsform ist bei dem oben erwähnten Adaptersystem wenigstens die Anbindungsplattform als 3D-druckbare Datei oder als in 3D-druckbare Daten umwandelbare Datensammlung ausgebildet.

Dies ermöglicht es, in besonders einfacher Art und Weise die Anbindungsplattform bei der Herstellung beispielsweise patientenindividueller Prothesenschafte als 3D-Daten mit einem Scan des Patientenstumpfes und einem hierum modellierten Prothesenschaft räumlich zu vereinigen und somit ein Gesamtdatenmodell aus Prothesenschafthülle und der Anbindungsplattform zu schaffen. Diese ist dann leicht einstückig im 3D-Druck ausdruckbar, so dass ein integrales Bauteil aus Prothesenschaft und Anbindungsplattform gebildet ist, ohne dass durch Eingießen oder durch Einlaminieren vorgefertigte Teile miteinander verbunden werden müssen.

In einer weiteren bevorzugten Ausführungsform ist somit die Anbindungsplattform integraler Teil einer 3D-druckbaren Datei oder als integraler Teil einer in 3D-druckbare Daten umwandelbaren Datensammlung des patientenindividuellen Prothesenschafts ausgebildet.

Weiterhin kann es vorteilhaft sein, die Anbindungsplattform mit einer Basisstruktur zu versehen, die einstückig, insbesondere im 3D-Druck einstückig hergestellt, mit dem Prothesenschaft ausgebildet ist.

Eine solche Basisstruktur kann in einfacher Art und Weise mit einer Hüllfläche des Prothesenschaftes, der als patientenindividueller 3D-Scan vorliegt, verschnitten werden, so dass in einfacher Art und Weise eine lückenlose Netzdatenstruktur herstellbar ist, die ohne Weiteres 3D-druckbar ist. Eine aufwändige Anbindung der Anbindungsplattform über Verschlussflächen an die 3D-Daten des Prothesenschaftes kann somit entfallen.

Die Anbindungsplattform, insbesondere deren Basisstruktur, ist dabei besonders bevorzugt übergroß ausgebildet, so dass in jedem Fall eine geschlossene Schnittkurve zwischen den 3D-Daten der Anbindungsplattform, insbesondere deren Basisstruktur und dem patientenindividuellen Prothesenschaft entsteht. "Überschüssige" Flächenanteile können in einem 3D-CAD-System nach dem Verschneiden ohne Weiteres gelöscht werden und stören bei der Herstellung des Prothesenschaftes dann nicht weiter.

Zweckmäßig kann es auch sein, dass die Anbindungsplattform wenigstens eine einen Formschluss mit der Adaptereinrichtung ausbildende Steckverbindungseinrichtung besitzt und insbesondere befestigungsmittellos mit der Adaptereinrichtung verbindbar ist, d. h. ohne zusätzliche Befestigungsmittel, wie z. B. Schrauben, Stifte, Nägel, Klemmen oder dergleichen mit der Adaptereinrichtung verbindbar ist.

Eine solche einen Formschluss mit der Adaptereinrichtung ausbildende Steckverbindungseinrichtung funktioniert somit unabhängig von weiteren Befestigungsmitteln und stellt somit in einfacher Art und Weise bei der Montage der Prothese einen einerseits leicht wechselbaren, andererseits nur durch Ein- und/oder Aufstecken verbindbare Austauschmöglichkeit der Adaptereinrichtung dar.

Des Weiteren kann es zweckmäßig sein, dass die Anbindungsplattform ein Befestigungslochraster besitzt, welches wenigstens teilweise mit einem Lochraster der Adaptereinrichtung korrespondiert und überlappt.

Hierdurch gelingt es beispielsweise durch die korrespondierende Ausgestaltung des Befestigungslochrasters und des Lochrasters eine einfache, sich überlappende Befestigungslochanordnung herzustellen, welche in einfacher Art und Weise durch Befestigungsmittel durchgriffen und verbunden werden kann.

Weiterhin kann es vorteilhaft sein, dass das Befestigungslochraster und das Lochraster mit einem Befestigungslochbild des Modulsystemelements, insbesondere des Schaftadapters korrespondiert, so dass ein sich wenigstens teilweise deckendes Dreifachlochbild entsteht, welches mit Befestigungsmitteln einfach durchdringbar ist.

Hierdurch kann in besonderes einfacher Art und Weise eine Befestigung des Modulsystemelements und des Adapterelements an der Anbindungsplattform erfolgen.

Zweckmäßiger Weise besitzt die Adaptereichrichtung einen Adapterflansch zum Zusammenwirken mit dem Modulsystemelement, insbesondere dem Schaftadapter derart, dass dieser hinsichtlich seiner Raumform auf das Befestigungslochbild und die entsprechenden Gegenanlageflächen des Schaftadapters angepasst ist, so dass der Schaftadapter, d. h. das Modulsystemelement ohne Nacharbeit direkt mit der Adaptereinrichtung zur Anlage kommen kann.

Weiterhin kann es vorteilhaft sein, dass eine Mittelachse M_{AF} des Adapterflansches koaxial oder versetzt zu einer Mittelachse M_{AP} der Anbindungsplattform ausgerichtet ist.

In diesen Anordnungsvarianten ist es in einfacher Art und Weise möglich, den Tragekomfort einer Prothese patientenindividuell anzupassen und zu optimieren.

Zweckmäßig ist auch, dass ein Befestigungslochabstand D_{BL} des Modulsystemelements, d. h. des Schaftadapters ein ganzzahliges Vielfaches eines Rastermaßes R1 der Anbindungsplattform ist.

Mit dieser Gestaltung gelingt eine einfache Befestigbarkeit des Modulsystemelements an der Anbindungsplattform.

Vorteilhaft kann auch sein, dass die Adaptereinrichtung mehrteilig aus wenigstens zwei Teilstücken ausgebildet ist, wobei jedes der Teilstücke mit der Anbindungsplattform in einer Richtung R winklig zur Mittelachse M_{AP}, insbesondere rechtwinklig zur Mittelachse M_{AP} formschlüssig mit der Anbindungsplattform verbindbar, insbesondere steckbar verbindbar ist.

Eine derartige mehrteilige Aufteilung der Adaptereinrichtung erleichtert die Montage der Adaptereinrichtung an der Anbindungsplattform erheblich und eröffnet die Möglichkeit, die Adaptereinrichtung beispielsweise durch Verbinden nur der beiden Teilstücke der Adaptereinrichtung fest und unlösbar mit der Anbindungsplattform zu verbinden.

Zudem wird durch den Formschluss eine hochfeste und belastbare Verbindung zwischen der Adaptereinrichtung und dem Prothesenschaft erzeugt, die wiederum eine feste und widerstandsfähige und hoch belastbare Anbindung des Schaftadapters, d. h. des Modulsystemelements verwirklichbar macht.

Weiterhin kann es zweckmäßig sein, dass die Teilstücke somit miteinander verbindbar, insbesondere verschraubbar sind.

Weiterhin kann es vorteilhaft sein, dass das Modulsystemelement mittels Befestigungselementen, d. h. z. B. der Schaftadapter mittels Befestigungselementen, welche die Adaptereinrichtung durchgreifen, unmittelbar mit der Anbindungsplattform verbindbar ist.

Hierdurch ist im fertigen Zustand einer Prothese gewährleistet, dass das Modulsystemelement, d. h. der Schaftadapter spielfrei, d. h. ohne Zwischenschaltung eines Adapterelements direkt mit dem Prothesenschaft, der ja bevorzugt einstückig ausgebildet ist, verbunden ist und somit eine hochfeste und gut belastbare Verbindung gewährleistet ist.

Im Folgenden wird die Erfindung anhand der Zeichnung beispielhaft näher erläutert. Es zeigen:
Figur 1: eine Vorderansicht auf ein Datenmodell eines patientenindividuell herzustellenden Prothesenschaftes beziehungsweise eines Prothesenschaftes, welcher eine Anbindungsplattform gemäß des erfindungsgemäßen Adaptersystems aufweist, wobei ein 3D-Datenmodell der Anbindungsplattform mit einem Scan-Modell des Prothesenschaftes verschnitten ist;
Figur 2: den Prothesenschaft gemäß Figur 1, wobei zusätzlich die Adaptereinrichtung und das Modulsystemelement, insbesondere der Schaftadapter, an dem Prothesenschaft montiert ist;
Figur 3a: eine isometrische Ansicht auf eine Ausführungsform der Anbindungsplattform zusammen mit einer Basisstruktur in einem räumlichen CAD-Modell;
Figur 3b: die Anbindungsplattform aus Figur 3a mit einer ersten Ausführungsform einer Adaptereinrichtung in teilmontierter Stellung;
Figur 3c: die Anbindungsplattform aus Figur 3a mit einer ersten Ausführungsform einer Adaptereinrichtung in fertigmontierter Stellung;
Figur 3d: die Anbindungsplattform aus Figur 3c zusätzlich aufweisend ein Modulsystemelement, z. B. einen Schaftadapter;
Figur 4a: das erfindungsgemäße Adaptersystem gemäß Figur 3c in einer Seitenansicht;
Figur 4b: eine Schnittansicht des erfindungsgemäßen Adaptersystems entlang der Linie A-A aus Figur 4a;
Figur 5: eine Draufsicht auf die Anbindungsplattform gemäß Figur 3a;
Figur 6: eine Draufsicht auf die erste Ausführungsform der Adaptereinrichtung;
Figur 7: eine Draufsicht auf eine zweite Ausführungsform der Adaptereinrichtung;
Figur 8: eine Draufsicht auf eine dritte Ausführungsform der Adaptereinrichtung.

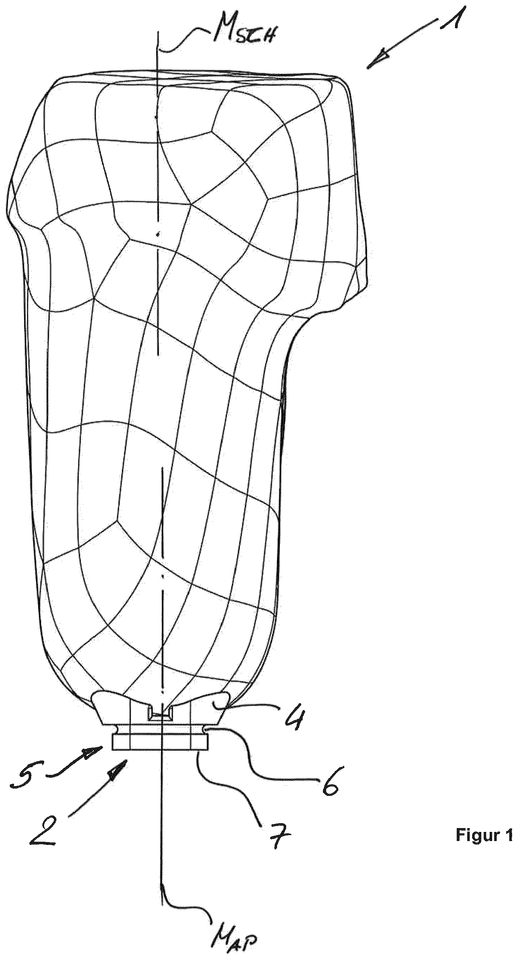

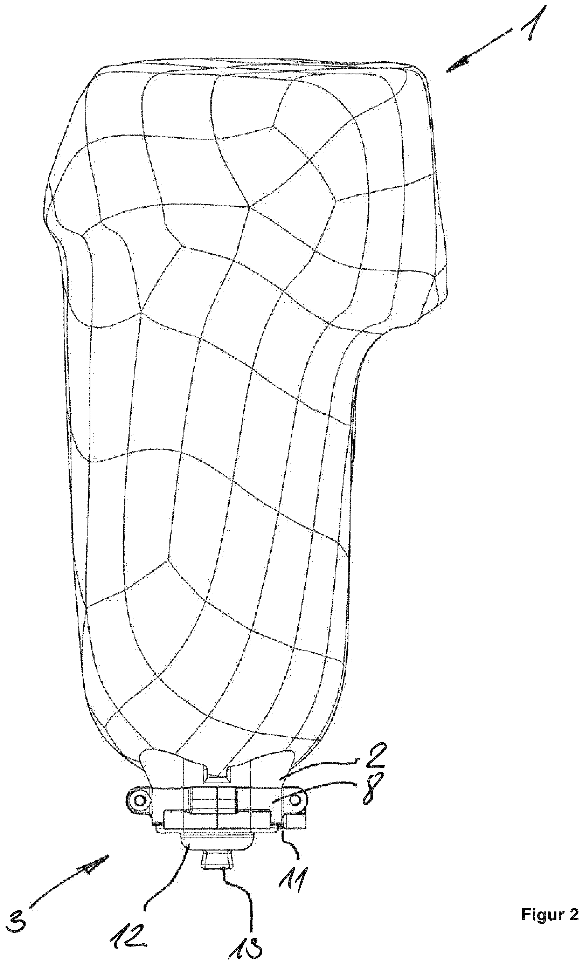

Figur 1 zeigt eine Vorderansicht auf ein Datenmodell eines patientenindividuell herzustellenden Prothesenschaftes 1 bzw. den Prothesenschaft 1 selbst, welcher eine Anbindungsplattform 2 gemäß des erfindungsgemäßen Adaptersystems 3 (siehe Figur 2) aufweist.

Ein 3D-Datenmodell der Anbindungsplattform 2 ist in der Darstellung gemäß Figur 2 mit einem Scan-Modell des Prothesenschaftes 1 verschnitten. Die Darstellung gemäß Figur 1 kann also sowohl eine Bildschirmansicht auf ein Datenmodell des Prothesenschaftes 1 zusammen mit der Anbindungsplattform 2 zeigen, oder auch eine schematisierte Ansicht auf einen tatsächlichen physischen Prothesenschaft 1, welcher die Anbindungsplattform 2 einstückig integriert besitzt.

Der Prothesenschaft 1 dient in üblicher Art und Weise zur Aufnahme eines Gliedmaßenstumpfes eines Patienten (nicht gezeigt). Beispielsweise kann der dargestellte Prothesenschaft 1 einen Oberschenkelstumpf (nicht gezeigt) aufnehmen. Die Anbindungsplattform 2 ist dabei erfindungsgemäß, d. h. abweichend vom Stand der Technik, nicht in den Prothesenschaft 1 einlaminiert oder mit diesem vergossen. Der dargestellte Prothesenschaft 1 wurde bereits als Datenscan-Modell mit dem Datenmodell der Anbindungsplattform 2 verschnitten und einstückig als 3D-gedrucktes Bauteil aus Prothesenschaft 1 und Anbindungsplattform 2 einstückig gedruckt.

Die Anbindungsplattform 2 weist hierzu eine Basisstruktur 4 auf, welche in den Prothesenschaft 1 übergeht. Die Anbindungsplattform 2 besitzt als Steckverbindungseinrichtung 5 eine z. B. umlaufende Nut 6, welche zwischen der Basisstruktur 4 und der Anbindungsplatte 7 umlaufend verläuft.

Für die weitere Beschreibung sei eine Mittelachse M_{AP} der Anbindungsplattform 2 definiert. Diese Mittelachse M_{AP} kann, muss aber nicht mit einer Mittelachse M_{SCH} des Prothesenschaftes 1 fluchten.

Die Mittelachse M_{SCH} des Prothesenschaftes 1 ist prinzipbedingt ohnehin eine lediglich gedachte Mittelachse M_{SCH}, da der Prothesenschaft 1 aufgrund der patientenindividuellen Anpassung an einen Gliedmaßenstumpf ein naturgemäß unsymmetrischer Körper ist. Die Mittelachse M_{SCH} des Prothesenschaftes 1 kann beispielsweise aus Schwerpunkten von Querschnittsflächen durch den Prothesenschaft 1 hergeleitet werden, wobei es dann sein kann, dass die Mittelachse M_{SCH} des Prothesenschaftes 1 gekrümmt verläuft.

Die Definition einer solchen Mittelachse M_{SCH} dient lediglich im Rahmen dieser Beschreibung zur Verdeutlichung von relativen Anordnungen von Bauteilen/Bestandteilen zueinander, falls dies zweckmäßig erscheint.

Das erfindungsgemäße Adaptersystem 3 umfasst neben der Anbindungsplattform 2 eine Adaptereinrichtung 8, welche, wie später beschrieben werden wird, die Nut 6 nutzend, auf die Anbindungsplattform 2 formschlüssig aufgesteckt ist. Die Adaptereinrichtung 8 bildet hierzu korrespondierend zur Nut 6 der Anbindungsplattform 2 (die Steckverbindungseinrichtung 5) eine Gegensteckverbindungseinrichtung 9 aus, die korrespondierend zur Nut 6 eine wulstartige Form besitzt, die in die Nut 6 einsteckbar ist.

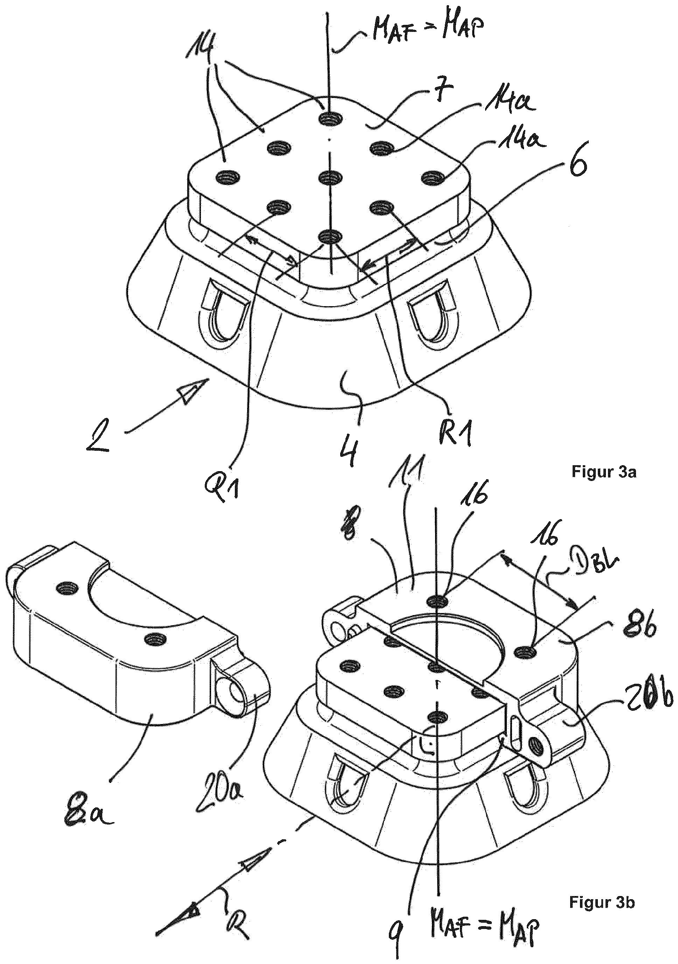

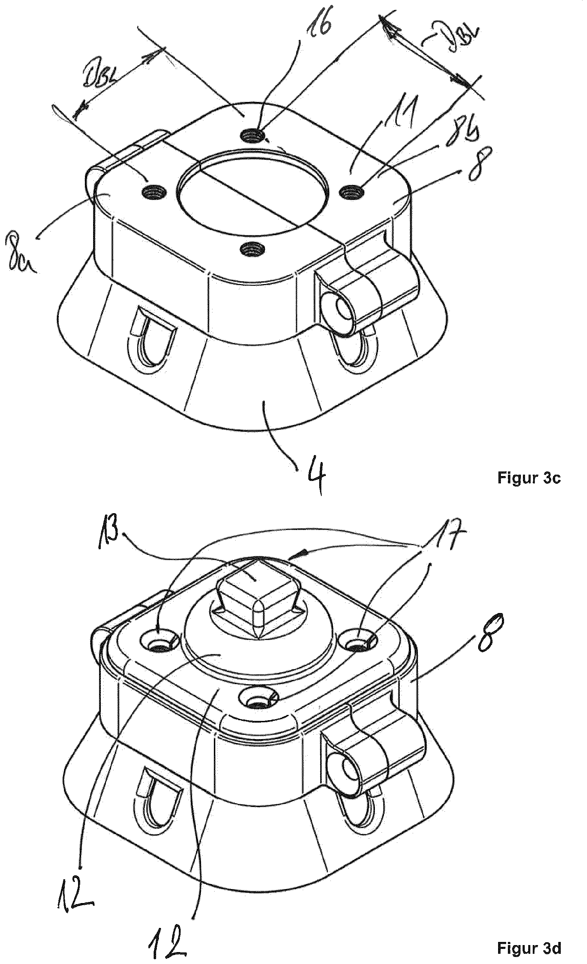

Eine solche Ausgestaltung ist auch in den Figuren 3a, 3b gezeigt.

Die Adaptereinrichtung 8 ist im Wege der Wechselwirkung der Steckverbindungseinrichtung 5 und der Gegensteckverbindungseinrichtung 9 auf die Anbindungsplattform 2 aufgesetzt und bildet einen freien Adapterflansch 11 aus.

Am Adapterflansch 11 ist ein Modulsystemelement 12, z. B. ein Schaftadapter befestigt, welches beispielsweise in bekannter Art und Weise einen umgekehrt pyramidenstumpfförmigen Anbindungsdom 13 zur Aufnahme von handelsüblichen Prothesenbauteilen, wie z. B. Prothesenkniegelenken, Prothesenrohrabschnitten und dergleichen besitzt.

Das Modulsystemelement 12 ist somit Teil eines handelsüblichen Modulsystems zum Herstellen von Prothesen, wobei derartige Prothesenbestandteile durch die umgekehrt pyramidenförmige Raumform 13 des Anbindungsdomes 13 in unterschiedlichen Winkeln und/oder Abständen relativ zum Anbindungsdom 13 bzw. relativ zum Modulsystemelement 12 befestigbar sind.

Das Adaptersystem 3 wird im Folgenden anhand der Figuren 3a bis 8 beispielhaft näher erläutert. Die vorbeschriebenen Figuren 1 und 2 zeigen die Anbindung des erfindungsgemäßen Adaptersystems 3 bzw. der Anbindungsplattform 2 des erfindungsgemäßen Adaptersystems 3 an dem Prothesenschaft 1.

In Figur 3a ist die Anbindungsplattform 2 in ihrer 3D-Datenstrukturausbildung gezeigt. Die Basisstruktur 4 hat eine vorgegebene Form und insbesondere eine trichterförmig aufgeweitete Raumform in einem derartigen Maße, dass ausreichend Flächendaten vorhanden sind, um die Anbindungsplattform 2 mit einem Scan-Modell des Prothesenschaftes 1 beispielsweise in einem Konstruktionsraum des CAD-Systems verschneiden zu können, ohne dass Flächenlücken oder dergleichen entstehen. Überschüssige Flächenabschnitte können in hergebrachter Art und Weise im CAD-System gelöscht werden, da sie zur weiteren Herstellung nicht benötigt werden.

Figur 3a zeigt außerdem die Anbindungsplatte 7, welche über die Nut 6 mit der Basisstruktur 4 verbunden ist. In der Anbindungsplatte 7 ist ein Befestigungslochraster 14 vorgesehen, welches im Beispiel gemäß Figur 3a aus einer Vielzahl von Befestigungslöchern 14a gebildet ist, die untereinander in zueinander senkrechten Richtungen ein Rastermaß R1 aufweisen. Die Rastermaße R1 sind bevorzugt in beiden aufeinander senkrecht stehenden Richtungen gleich.

Die Anbindungsplattform 2 (vgl. Figur 3b) ist aus zwei Anbindungsplattformhälften 8a, 8b gebildet, welche derart eingerichtet und ausgebildet sind, dass sie in einer Richtung R unter Zusammenwirkung der Gegensteckverbindungseinrichtung 9 und der Adaptereinrichtung 8 auf die Anbindungsplattform 2 aufsteckbar ist.

Die Adaptereinrichtung 8, 8a, 8b weist dabei ein Lochraster 16 auf, welches mit einem Befestigungslochbild 17 (vgl. Figur 3d) des Modulsystemelements 12 korrespondiert.

Die Richtung R kann bevorzugt rechtwinklig zur Mittelachse M_{AF} verlaufen oder spitzwinklig zu dieser angeordnet sein.

Das Lochraster 16 besitzt einen Befestigungslochabstand D_{BL}, der in beiden Richtungen korrespondierend zum Rastermaß R1 ein ganzzahliges Vielfaches des Rastermaßes R1 beträgt, so dass das Lochraster 16 mit wenigstens einigen der Löcher 14a fluchtet. Im Ergebnis fluchten somit Löcher des Befestigungslochbildes 17 des Modulsystemelements 12 mit den Löchern des Lochrasters 16 der Adaptereinrichtung 8, 8a, 8b und wenigstens einer Auswahl der Löcher 14a des Befestigungslochrasters 14.

Dies ermöglicht es, das Modulsystemelement 12 beispielsweise mittels Schrauben, die die Löcher des Befestigungslochbildes 17 durchgreifen, die Löcher des Lochrasters 16 durchgreifend mit der Anbindungsplattform 2 bzw. deren Löchern 14a des Befestigungslochrasters 14 zu verbinden. Eine derartige durchgreifende Befestigung bewirkt einen besonders festen Verbund zwischen dem Modulsystemelement 12, der Adaptereinrichtung 8 und der Anbindungsplattform 2. Zusätzlich kann, wie gezeigt in den Figuren 3b bis 3d, die Adaptereinrichtung 8 geteilt ausgebildet sein, so dass Teilstücke 8a, 8b gebildet sind.

Zur Befestigung der Teilstücke 8a, 8b miteinander sind zweckmäßigerweise Schraubvorsprünge 20a, 20b vorgesehen, wobei durch Einschrauben einer Schraube die Teilstücke 8a, 8b in Aufsteckrichtung R miteinander verbunden werden, so dass ein fester unlösbarer, formschlüssiger Verbund entsteht, da durch ein Verschrauben der beiden Teilstücke 8a, 8b ein zerstörungsfreies Trennen des Formschlusses zwischen der Anbindungsplattform 2 und der Adaptereinrichtung 8 nicht mehr möglich ist. Die umlaufende Nut 6 in Zusammenwirkung mit der wulstartigen Steckverbindungseinrichtung 5 bildet einen solchen zerstörungsfrei unlösbaren Formschluss.

In der Ausführungsform gemäß der Figur 3a bis 3d fluchtet eine Mittelachse M_{AF} des Adapterflansches 11 mit der Mittelachse M_{AP} der Anbindungsplattform, so dass das Adaptersystem entlang der Mittelachse M_{AP}/M_{AF} aufeinanderfolgend ausgerichtet ist.

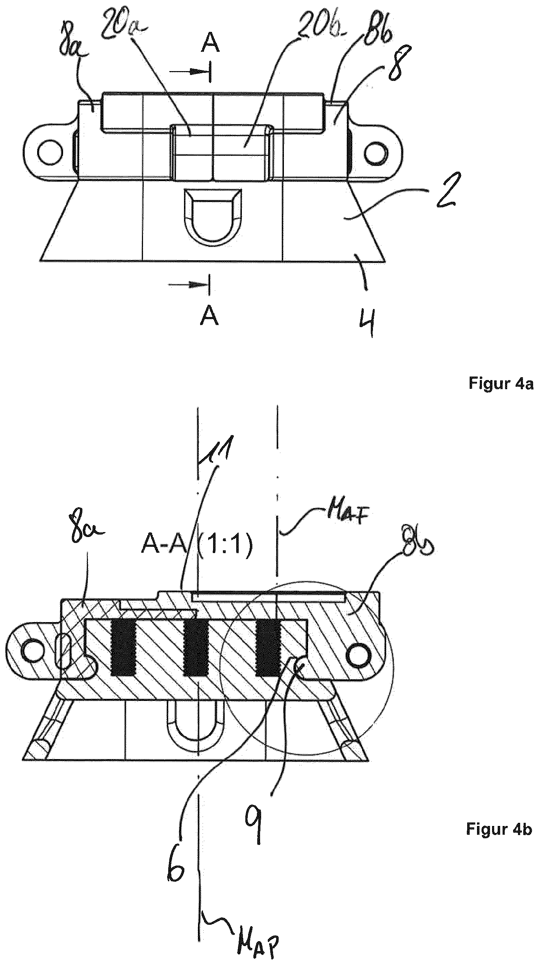

Figur 4a zeigt eine Anbindungsplattform 2 mit der aufgesetzten Adaptereinrichtung 8 in einer Seitenansicht. Ebenfalls sichtbar sind die Schraubvorsprünge 20a, 20b der Teilstücke 8a, 8b. Entlang der Schnittlinie A-A ist ein Schnitt gelegt, der in Figur 4b maßstäblich dargestellt ist. Ebenfalls dargestellt ist die wulstartige Gegensteckverbindungseinrichtung 9, welche mit der Nut 6 zusammenwirkt.

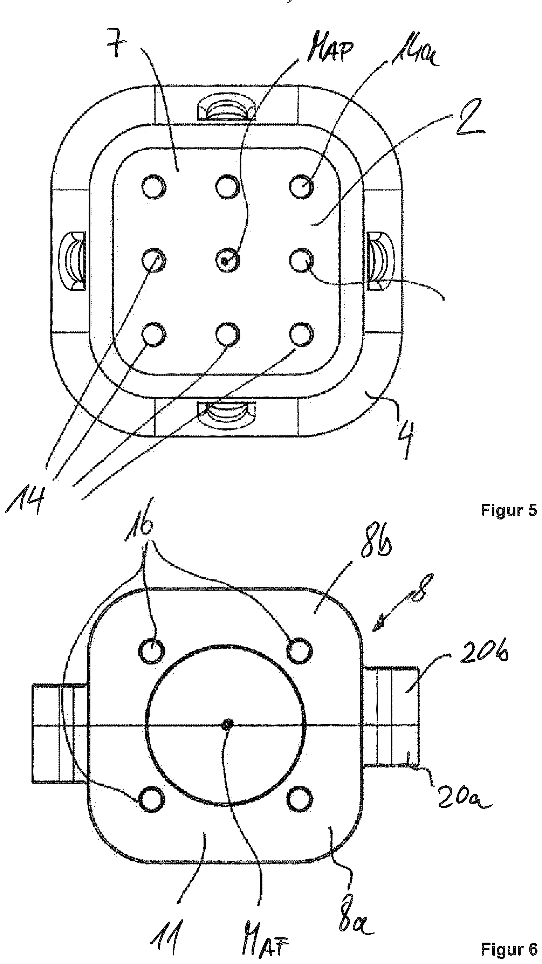

Bei dem Ausführungsbeispiel gezeigt in den Figuren 4a, 4b ist ein Fall gezeigt, bei dem die Mittelachse M_{AP} der Anbindungsplattform 2 versetzt parallel zu der Mittelachse M_{AF} des Adapterflansches 11 angeordnet ist. Eine derartige zueinander relativ versetzte Anordnung wird nachfolgend anhand der Figuren 5 bis 8 weiter verdeutlicht.

Figur 5 zeigt nochmals eine Draufsicht auf die Anbindungsplattform 2 des Adaptersystems 3 mit den Löchern 14a, welche im Befestigungslochraster 14 angeordnet sind.

Die Mittelachse M_{AP} ist in Figur 5 als Punkt dargestellt und erstreckt sich senkrecht zur Zeichenebene durch das mittlere Loch 14a des Befestigungslochrasters 14.

Die Basisstruktur 4 ist radial von der Mittelachse M_{AP} nach außen abgehend durchweg größer als die Anbindungsplatte 7 und überragt diese in der Radialrichtung R.

Figur 6 zeigt eine erste Ausführungsform der Adaptereinrichtung 8 mit den Teilstücken 8a, 8b und den Schraubvorsprüngen 20a, 20b in einer Draufsicht.

Die Löcher 14a des Lochrasters 16 sind erkennbar. Der Adapterflansch 11 ist die freie Oberfläche der Adaptereinrichtung 8.

Die Mittelachse M_{AF} ist in der Darstellung gemäß Figur 6 ebenfalls als Punkt dargestellt und verläuft senkrecht zur Zeichenebene. Wenn die Adaptereinrichtung 8 gemäß Figur 6 mit der Anbindungsplattform 2 gemäß Figur 5 verbunden wird, fluchten die Mittelachsen M_{AP} und M_{AF}.

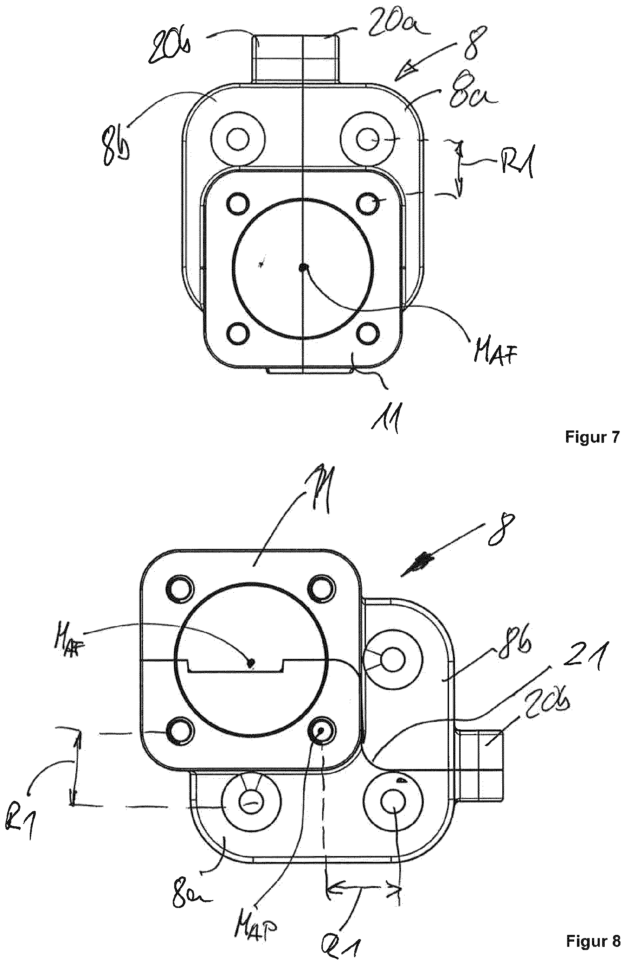

Eine abgewandelte Ausführungsform der Adaptereinrichtung 8 zeigt Figur 7. Die Mittelachse M_{AF} des Adapterflansches 11 ist im Falle einer Montage der Ausführungsform der Adaptereinrichtung 8 gemäß Figur 7 um ein Rastermaß R1 versetzt zur Mittelachse M_{AP} der Anbindungsplattform 2 angeordnet. Somit kann das Modulsystemelement 12 (nicht gezeigt in Figur 7) versetzt zur Mittelachse M_{AP} der Anbindungsplattform 2 an der Adaptereinrichtung 8 angebracht werden. Eine derartige versetzte Montage kann zum Ausbalancieren einer Prothese hinsichtlich auftretender Drehmomente bei Belastung sinnvoll sein. Die Adaptereinrichtung 8 gemäß Figur 7 ist dabei in einer Richtung um das Rastermaß R1 versetzt zur Mittelachse M_{Ap} angeordnet.

Eine weitere Ausführungsform der Adaptereinrichtung 8 gemäß Figur 8 weist ebenfalls einen Adapterflansch 11 auf, der versetzt zur Mittelachse M_{AP} der Anbindungsplattform 2 ist. In der Ausführungsform gemäß Figur 8 ist der Adapterflansch 11 in zwei zueinander senkrechten Richtungen um das Maß R1 versetzt zur Mittelachse M_{AP} angeordnet. Mit dieser Ausführungsform ist eine außermittige Anordnung weiterer Prothesenbestandteile einfach möglich.

Durch die in zwei Richtungen versetzte Anordnung des Adapterflansches 11 bezüglich der Mittelachse M_{AP} der Anbindungsplattform 2 ergibt sich eine gestufte Trennfuge 21, die es ermöglicht, die Teilstücke 8a, 8b in etwa gleich schwer zu gestalten, was zu etwa gleichen Herstellzeiten im Wege des 3D-Druckes führt.

In besonders bevorzugter Art und Weise ist das erfindungsgemäße Adaptersystem 3 bestehend aus der Anbindungsplattform 2 und der Adaptereinrichtung 8 als 3D-druckbares Datenmodell oder als in 3D-druckbare Daten umwandelbare Datensammlung ausgebildet. Eine Anpassung derartiger Datensammlungen/Datenmodelle an patientenindividuell eingescannte Prothesenschäfte 1 kann in einfacher Art und Weise in einem CAD-Konstruktionsraum eines geeignet ausgestatteten Computersystems vorgenommen werden. Somit kann die Anbindungsplattform 2 lückenlos in ein CAD-Modell (räumliches Datenmodell) des Prothesenschaftes 1 eingebunden werden und in besonders bevorzugten Ausgestaltung einstückig beim Ausdrucken des Prothesenschaftes 1 mitgedruckt werden. Somit ergibt sich ein integral ausgebildetes, einstückiges Bauteil, welches in einem einzigen Arbeitsgang, z. B. dem 3D-Druck herstellbar ist.

Zweckmäßiger Weise liegen auch die Teilstücke 8a, 8b der Adaptereinrichtung als 3D-Datenmodell oder in 3D-Daten umwandelbare Datensammlung vor. Derartige Teilstücke 8a, 8b können aber auch zweckmäßiger Weise vorgefertigt sein und in einem anderen Herstellverfahren, z. B. dem Spritzgussverfahren gefertigt sein.

Wie oben beschrieben, ist es besonders vorteilhaft, die Anbindungsplattform 2 datenmäßig in ein Datenmodell des Prothesenschaftes zu integrieren, um einen einstückigen, 3D-druckbaren Prothesenschaft 1 zu erhalten. Selbstverständlich ist es aber auch denkbar, die Anbindungsplattform 2 in herkömmlicher Art und Weise durch Einlaminieren oder Eingießen mit einem vorgefertigten Prothesenschaft 1 zu verbinden. Die erfindungsgemäßen Vorteile werden allerdings in besonderem Maße mittels der Ausbildung als 3D-druckbare Daten oder in 3D-druckbare Daten umwandelbare Datensammlung erreicht.

### Bezugszeichenliste

- 1: Prothesenschaft
- 2: Anbindungsplattform
- 3: Adaptersystem
- 4: Basisstruktur
- 5: Steckverbindungseinrichtung
- 6: Nut
- 7: Anbindungsplatte
- 8: Adaptereinrichtung
- 8a, 8b: Teilstücke
- 9: Gegensteckverbindungseinrichtung

- 11: Adapterflansch
- 12: Modulsystemelement
- 13: Anbindungsdom
- 14: Befestigungslochraster
- 14a: Löcher
- 16: Lochraster
- 17: Befestigungslochbild

- 20a, 20b: Schraubvorsprünge
- 21: Trennfuge

- M_{AP}: Mittelachse der Anbindungsplattform
- M_{AF}: Mittelachse der Anbindungsplattform
- M_{SCH}: Mittelachse des Schaftes
- D_{BL}: Befestigungslochabstand
- R1: Rastermaß
- R: Richtung

## Patentansprüche

1. Adaptersystem zwischen einem Prothesenschaft (1) und handelsüblichen Modulsystemen aufweisend:
- eine Anbindungsplattform (2)
- eine an der Anbindungsplattform (2) befestigbare Adaptereinrichtung (8), wobei
- wenigstens ein Modulsystemelement (12) wenigstens an der Adaptereinrichtung (2) befestigbar ist.

2. Adaptersystem nach Anspruch 1, **dadurch gekennzeichnet, dass** wenigstens die Anbindungsplattform (2) als 3D-druckbare Datei oder als in 3D-druckbare Daten umwandelbare Datensammlung ausgebildet ist.

3. Adaptersystem nach Anspruch 1 oder 2, **dadurch gekennzeichnet, dass** die Anbindungsplattform (2) integraler Teil einer 3D-druckbaren Datei oder als integraler Teil einer in 3D-druckbare Daten umwandelbare Datensammlung des patientenindividuellen Prothesenschafts (1) ist.

4. Adaptersystem nach einem der vorhergehenden Ansprüche, **dadurch gekennzeichnet, dass** die Anbindungsplattform (2) eine Basisstruktur (4) aufweist, die einstückig, insbesondere im 3D-Druck einstückig hergestellt mit dem Prothesenschaft (1) ausgebildet ist.

5. Adaptersystem nach einem der vorhergehenden Ansprüche, **dadurch gekennzeichnet, dass** die Anbindungsplattform (2) wenigstens eine einen Formschluss mit der Adaptereinrichtung (8) ausbildende Steckverbindungseinrichtung (5) besitzt und insbesondere befestigungsmittelos mit der Adaptereinrichtung verbindbar ist.

6. Adaptersystem nach einem der vorhergehenden Ansprüche, **dadurch gekennzeichnet, dass** die Anbindungsplattform (2) ein Befestigungslochraster (14) besitzt, welches wenigstens teilweise mit einem Lochraster (16) der Adaptereinrichtung (8) korrespondiert und überlappt.

7. Adaptersystem nach einem der vorhergehenden Ansprüche, **dadurch gekennzeichnet, dass** das Befestigungslochraster (14) und das Lochraster (16) mit einem Befestigungslochbild (17) des Modulsystemelements (12) korrespondiert.

8. Adaptersystem nach einem der vorhergehenden Ansprüche, **dadurch gekennzeichnet, dass** die Adaptereinrichtung (8) einen Adapterflansch (11) zum Zusammenwirken mit dem Modulsystemelement (12) besitzt.

9. Adaptersystem nach einem der vorhergehenden Ansprüche, **dadurch gekennzeichnet, dass** eine Mittelachse (M_{AF}) des Adapterflansches (11) koaxial oder versetzt zu einer Mittelachse (M_{AP}) der Anbindungsplattform (2) ausgerichtet ist.

10. Adaptersystem nach einem der vorhergehenden Ansprüche, **dadurch gekennzeichnet, dass** ein Befestigungslochabstand (D_{BL}) des Modulsystemelements (12) ein ganzzahliges Vielfaches eines Rastermaßes (R1) der Anbindungsplattform (2) ist.

11. Adaptersystem nach einem der vorhergehenden Ansprüche, **dadurch gekennzeichnet, dass** die Adaptereinrichtung (8) mehrteilig aus wenigstens zwei Teilstücken (8a; 8b) ausgebildet ist, wobei jedes der Teilstücke (8a; 8b) mit der Anbindungsplattform (2) in einer Richtung (R) winklig zur Mittelachse (M_{AP}), insbesondere rechtwinklig zur Mittelachse (M_{AP}) formschlüssig mit der Anbindungsplattform (2) verbindbar, insbesondere steckbar verbindbar ist.

12. Adaptersystem nach einem der vorhergehenden Ansprüche, **dadurch gekennzeichnet, dass** die Teilstücke (8a; 8b) miteinander verbindbar, insbesondere verschraubbar sind.

13. Adaptersystem nach einem der vorhergehenden Ansprüche, **dadurch gekennzeichnet, dass** das Modulsystemelement (12) mittels Befestigungselementen, welche die Adaptereinrichtung (8) durchgreifen, unmittelbar mit der Anbindungsplattform (2) verbindbar ist.

14. Adaptereinrichtung für ein Adaptersystem (3) nach einem der vorangegangenen Ansprüche, wobei die Adaptereinrichtung (8) als 3D-druckbare Dateien der Teilstücke oder als in 3D-druckbare Daten umwandelbare Daten der Teilstücke (8a; 8b) vorliegt oder als physischer Körper aus den Teilstücken (8a; 8b) ausgebildet ist.

15. Prothesenschaft eingerichtet und ausgebildet zum Zusammenwirken mit wenigstens der Adaptereinrichtung (8) nach Anspruch 14 oder aufweisend die Anbindungsplattform (2) des Adaptersystems (3) nach einem der Ansprüche 1 bis 13.

16. Prothese aufweisend wenigstens einen Prothesenschaft (1) nach Anspruch 15 und/oder ein Adaptersystem (3) nach einem der Ansprüche 1 bis 13.
